Europäisches Patentamt

European Patent Office

Office européen des brevets

(19)

(11) EP 0 872 244 B1

(12) **FASCICULE DE BREVET EUROPEEN**

(45) Date de publication et mention
de la délivrance du brevet:
**07.07.2004 Bulletin 2004/28**

(51) Int Cl.⁷: **A61K 35/78**, A61P 17/00

(21) Numéro de dépôt: **98400904.3**

(22) Date de dépôt: **10.04.1998**

(54) **Composition cosmétique ou pharmaceutique contenant un extrait d'Andiroba**

Andiroba Extrakt enthaltende kosmetische oder pharmazeutische Zusammensetzung

Cosmetic or pharmaceutical composition containing an Andiroba extract

(84) Etats contractants désignés:
**AT BE CH DE DK ES FI FR GB GR IE IT LI LU MC NL PT SE**

(30) Priorité: **14.04.1997 FR 9704550**

(43) Date de publication de la demande:
**21.10.1998 Bulletin 1998/43**

(73) Titulaire: **LABORATOIRES DE BIOLOGIE VEGETALE YVES ROCHER
56201 La Gacilly (FR)**

(72) Inventeurs:
• **Rouillard, Françoise
91160 Longjumeau (FR)**
• **Crepin, Juliette
75006 Paris (FR)**

• **Saintigny, Gaelle
75018 Paris (FR)**

(74) Mandataire: **Bernasconi, Jean Raymond et al
c/o Cabinet Lavoix,
2, Place d'Estienne d'Orves
75441 Paris Cedex 09 (FR)**

(56) Documents cités:
• **DATABASE WPI Section Ch, Week 8831 Derwent Publications Ltd., London, GB; Class B04, AN 88-213108 XP002052075 & BR 8 605 739 A (GEROLANO D Q) , 28 juin 1988**
• **MATTHIAS L. A. HAMMER ET AL.: "TAPPING AN AMAZ NIAN PLETHORA: FOUR MEDICINAL PLANTS OF MARAJ ISLAND, PAR (BRAZIL)" JOURNAL OF ETHNOPHARMACOLOGY, vol. 40, no. 1, septembre 1993, pages 53-75, XP002052074**

**Description**

L'invention concerne le domaine cosmétique et pharmaceutique, notamment le traitement de la cellulite.

L'invention a plus particulièrement pour objet l'utilisation d'un extrait lipidique d'Andiroba pour la préparation d'une composition pharmaceutique ou cosmétique destinée au traitement de la cellulite.

L'Andiroba (Carapa guianens is Aubl.) est un arbre qui appartient à la famille des Méliacées. Originaire de la forêt tropicale de l'Amazonie, sa croissance peut atteindre 25 mètres, le tronc est terminé par une large frondaison de feuilles et branches, ses fruits sont de grosses capsules contenant 7 à 8 graines de formes variables.

Les graines de l'Andiroba renferment une amande, qui représente 72 à 74 % du poids de la graine et qui est riche en matière grasse (de l'ordre de 60-66 %).

L'extrait lipidique que l'on peut tirer de ces amandes est utilisé traditionnellement par voie externe pour ses propriétés anti-inflammatoires, pour calmer les douleurs musculaires et articulaires, traiter les piqûres d'insectes ou pour tonifier les cheveux et chasser les poux. Il est aussi utilisé en tant que désinfectant ou cicatrisant de la peau. Par voie interne, c'est un tonique général.

Les auteurs de la présente invention ont mis en évidence que l'extrait lipidique d'Andiroba possède un effet inhibiteur de la glucose-6-phosphate deshydrogènase et de surcroît un effet inhibiteur de la conversion adipocytaire.

L'invention concerne ainsi l'utilisation d'un extrait lipidique d'Andiroba pour l'inhibition de la glucose-6-phosphate deshydrogénase (G6PDH).

L'invention concerne également l'utilisation d'un extrait lipidique de l'invention pour l'obtention d'un effet inhibiteur de la conversion adipocytaire.

Grâce à cet effet sur la conversion adipocytaire, l'extrait lipidique d'Andiroba selon l'invention trouve des applications particulièrement intéressantes pour les utilisations dans une composition cosmétique ou pour la préparation d'une composition pharmaceutique destinée à la régulation des mécanismes impliqués dans la cellulite afin d'obtenir un effet anti-cellulite.

Par traitement de la cellulite au sens de la présente invention, on entend un traitement essentiellement préventif ou destiné à limiter l'expansion d'une cellulite établie.

En effet, l'extrait huileux d'Andiroba n'a pratiquement pas d'action sur la cellulite établie, mais agit sur l'inhibition de la différenciation des préadipocytes en adypocytes, limite donc la possibilité d'installation de la cellulite.

Au coeur du métabolisme des graisses, sous l'épiderme, se trouve le tissu adipeux, qui représente 15 à 20 % du poids corporel. Le tissu adipeux est formé d'une matrice conjonctive qui renferme les adipocytes (gestionnaires énergétiques, via les triglycérides) et des fibroblastes spécialisés ou préadipocytes.

Si les préadipocytes, précurseurs des adipocytes ont une morphologie allongée, caractéristique des fibroblastes, les adipocytes au terme de leur différenciation sont des cellules sphériques dont l'espace intercellulaire est comblé par une large vécicule remplie de triglycérides. Le phénomène de différenciation est sous la dépendance de facteurs hormonaux, facteurs de croissances et prostaglandines.

Dans le domaine de l'invention, c'est-à-dire le traitement de la cellulite, les inventeurs ont eu une approche originale de la minceur, qui a consisté à contrôler le phénomène de différenciation des préadipocytes en adipocytes matures.

Une des enzymes clef de cette différentiation est la glucose-6-phosphate deshydrogénase qui régule la voie des pentoses, et se trouve répartie dans différents tissus de l'organisme, tels : le foie, les glandes mammaires, le tissu adipeux. Elle est cytoplasmique et active sous forme dimérique. Elle catalyse en présence de NADP⁺, l'oxydation du βD-glucose-6-phosphate en 6 phosphoglucopyrano-1,5-lactone et NADPH. La réaction est thermodynamiquement réversible, mais elle est rendue irréversible par l'hydrolyse excessivement rapide du de la 6-phosphoglucopyrano-1,5-lactone, à pH > 6,4

Dans le tissu adipeux, l'enzyme en question permet ainsi de générer : NADPH, source primaire du pouvoir réducteur dans la synthèse des acides gras et du ribose 5 phosphate, source de ribose pour la biosynthèse de métabolites comme l'ARN, l'ATP, le Coenzyme A.

L'extrait d'Andiroba selon l'invention est obtenu à partir des amandes de Capara guianensis extraites des noix.

Les principaux constituants actuellement identifiés dans cet extrait lipidique sont :

- des méliacines et des terpènes oxygénés proches des quassinoïdes ;
- des acides gras : acide palmitique (31 %), acide stéarique (7 %), acide arachidique (2 %), acide hexadécénoïque (1 %), acide oléique (49,3 %), acide linoléique (9 %), acide linolénique (0,7 %).

Les extraits lipidiques d'Andiroba peuvent être obtenus par un traitement à chaud après réduction des amandes en pâte, puis un passage sous presse. L'extrait obtenu dans ce cas, est un liquide visqueux, il est décanté puis éventuellement filtré.

Une autre possibilité est d'extraire la fraction huileuse de l'amande concassée au moyen d'un solvant apolaire, de type hexane, les proportions de solvant par rapport à la matière première étant de préférence de 1 à 1,25 litres par kilo de matière. On effectue avantageusement l'extraction à l'aide d'un solvant chauffé jusqu'à une température inférieure à 60°C, en contrôlant le débit du solvant et le nombre de lavages successifs. L'extrait obtenu dans ce cas est distillé afin de séparer la fraction lipidique, du solvant qui a servi à son extraction.

De nouvelles technologies connues de l'homme du métier, comme par exemple l'utilisation de gaz supercritique, pourront aussi être utilisées pour obtenir l'extrait lipidique d'Andiroba.

Les extraits obtenus selon l'invention peuvent contenir 0,05 % de BHT en poids à titre d'anti-oxydant.

On décrira ci-après des exemples de mise en évidence des propriétés de l'extrait lipidique d'Andiroba de l'invention.

## EXEMPLE 1

I. Mise en évidence *in vitro* de l'effet inhibiteur de la glucose-6-phosphate deshydrogènase.

Un test *in vitro* a été développé au moyen d'une G6PDH isolée à partir de levure de boulanger et commercialisée. Cette technique colorimétrique permet d'identifier et de sélectionner des inhibiteurs potentiels de la différenciation des préadipocytes en adipocytes.

L'enzyme (G6PDH) est mise en présence de D-glucose-6-phosphate et de nicotinamide adénine dinucléotide phosphate (NADP$^+$) L'activité enzymatique est mesurée par la réduction du NADP$^+$ en NADPH et par l'oxydation du D-glucose-6-phosphate en 6-phosphoglucopyrano-1,5 lactone. Le nicotinamide adénine phosphate réduit (NADPH) réagit ensuite avec un système phénazine méthosulfate/ Bleu de thiazolyle (PMS/MTT) pour donner un complexe coloré qui absorbe à 560 nm. (Richard W. Geisler, Alyn M. McClure and Robert J. Hansen, Biochimica et Biophysica Acta, 327, 1973, 1-10).

Pour chaque cinétique, une vitesse moyenne est mesurée

Le rapport des vitesses moyennes X sur la vitesse moyenne du témoin activité, multiplié par 100, permet de calculer un pourcentage d'inhibition.

Le volume, de principe actif testé, nécessaire pour obtenir une inhibition de 50 % est déterminé graphiquement.

L'effet inhibiteur d'un principe actif est défini par rapport à un inhibiteur de référence : le palmitoyl CoA.

$$\text{Effet inhibiteur (U.A)} = \frac{X \ \mu\text{l Palmitoyl CoA x [Palmitoyl CoA]}}{y \ \mu\text{l Solution X x [Solution X]}} \text{x } 100$$

L'extrait lipidique d'Andiroba obtenu selon l'un des procédés décrit ci-dessus, est solubilisé dans du diméthyl sulfoxide (DMSO) et testé *in vitro* sur l'activité de la glucose-6-phosphate deshydrogénase

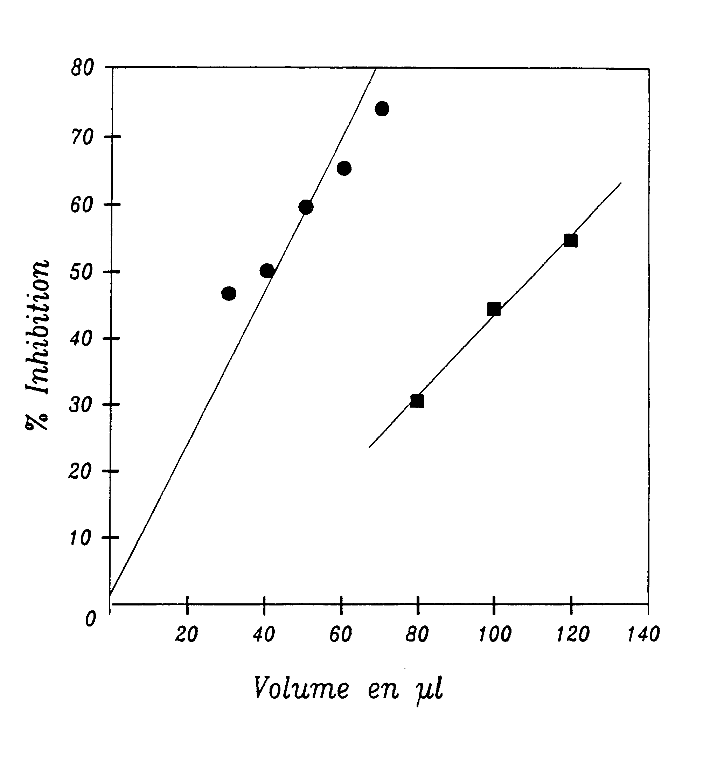

Les résultats sont illustrés à la figure annexée.

L'effet inhibiteur est de 1,9 à 2,8 (Unités Arbitraires), c'est-à-dire que l'extrait lipidique d'Andiroba est 30 à 50 fois moins actif que le palmitoyl CoA. Il possède cependant un potentiel inhibiteur intéressant, qui permet d'envisager son utilisation comme inhibiteur de la différenciation des préadipocytes en adipocytes.

## EXEMPLE 2

II. Mise en évidence *in vitro* de l'effet inhibiteur de la différenciation adipocytaire.

L'étude est réalisée sur une lignée cellulaire 3T3L1, qui sous certaines conditions de culture est capable de se différencier en accumulant des vésicules contenant des triglycérides. Le stade de différenciation adipocytaire est proportionnel à l'activité de la G3POH, qui est le marqueur spécifique de cette différenciation.

Les cellules sont d'abord amenées à confluence en utilisant un milieu de prolifération (DMEM + 10 % de sérum de veau foetal) Puis la différenciation cellulaire est induite en incorporant dans ce milieu de culture de l'insuline, de la dexaméthasone et de l'isobutyl méthyl xanthine. L'actif à tester est introduit au moment de la différenciation. Son effet est proportionnel à la quantité de l'activité G3PDH cellulaire, exprimée par mg de protéines.

L'activité G3PDH est déterminée *in vitro* sur le surnageant d'un broyat cellulaire : la G3PDH en présence de β-nicotinamide adénine dinucléotide, forme réduite (NADH$_2$), transforme le dihydroxyacétone phosphate (DHAP) en glycérol phosphate. La disparition de NADH$_2$ est reliée à l'activité GP3PDH et est suivie au spectrophotomètre à 340 nm.

Si l'extrait lipidique d'Andiroba, solubilisé à 0,02 % dans du DMSO, est introduit au moment de l'induction de la différenciation, l'expression résultante de l'activité G3PDH du surnageant cellulaire, est inhibée de 25 à 50 % par rapport à l'expression de l'activité G3PDH du surnageant cellulaire témoin (sans actif).

Les extraits lipidiques d'Andiroba, obtenus selon l'invention, inhibiteurs de G6PDH et de surcroît, inhibiteurs de la conversion adipocytaire, sont donc utilisables en cosmétique de soins ou en pharmacie, pour aider la régulation des mécanismes de contrôle de la cellulite.

Leur mode d'administration est réalisé par voie topique

Les extraits d'Andiroba selon l'invention peuvent être utilisés tels quels, vectorisés, microencapsulés, en association avec un mélange d'excipients tels que huiles végétales, minérales, cires végétales ou minérales, silicones, alcools et acides gras, agents tensio actifs, etc., ou en association avec d'autres extraits végétaux ou molécules d'origine naturelle.

Les compositions couvrent toutes les formes cosmétiques ou pharmaceutiques, du type émulsions simples H/E ou E/H, émulsions multiples ou microémulsions, gels aqueux ou lipophiles, huiles, spray, sticks, etc.

Les exemples suivants de composition contenant un extrait lipidique d'Andiroba en tant que principe actif, illustrent la présente invention.

Les pourcentages sont donnés en poids.

## EXEMPLE 3 :

### Composition sous forme de gel aqueux :

| | |
|---|---|
| Acide polyacrylique | 0,5 - 1,2 % |
| Gomme xanthane | 0,3 - 0,5 % |
| Eau | qsp 100 % |
| Humectants | 5,0 - 10,0 % |
| Extrait lipidique d'Andiroba | 0,01 - 5,0 % |
| Extraits autres | 0 - 20,0 % |
| Ethanol | 5,0 - 30,0 % |
| Parfum | 0,2 % |
| NaOH | 0,1 - 0,6 % |
| Colorant | 0,05 - 0,5 % |

## EXEMPLE 4 :

### Composition sous forme d'émulsion H/E (lait ou crème) :

| | |
|---|---|
| Eau | qsp 100 % |
| Conservateurs | qsp |
| Humectants | 5,0 - 10,0 % |
| Gomme xanthane | 0,1 - 0,3 % |
| Copolymère acrylique/acrylate | 0,1 - 0,5 % |
| Acide stéarique 10 OE | 3,0 % |
| Stéarate de sorbitan | 2,0 % |
| Sorbitan stéarate 20 OE | 3,0 % |
| Alcool cétylstéarique | 0 - 1,5 % |
| Extrait lipidique d'Andiroba | 0,5 - 25,0 % |
| Acétate de tocophérol | 0,1 % |
| Silicones | 2,0 - 7,0 % |
| Gel de polyacrylamide | 0 - 2,0 % |
| Autres extraits | 0 - 20,0 % |
| Parfum | 0,3 % |

### EXEMPLE 5 :

### Composition sous forme de lotion :

| Eau | qsp 100 % |
|---|---|
| Humectants | 3,0 - 10,0 % |
| Conservateurs | qsp |
| Alcool | 1,0 - 7,0 % |
| Solubilisant | 0 - 15,0 % |
| Extrait lipidique d'Andiroba | 0,01 - 10,0 % |
| Autres extraits | 0 - 20,0 % |
| Parfum | 0,05 - 0,2 % |

### EXEMPLE 6 :

### Composition sous forme d'huile corporelle:

| Huile minérale | qsp 100 % |
|---|---|
| Acétate de tocophérol | 0,1 % |
| Extrait lipidique d'Andiroba | 1,0 - 25,0 % |
| Huile végétale | 1,0 - 25,0 % |
| Extraits | 0 - 10,0 % |
| Parfum | 0,3 - 1,0 % |

### EXEMPLE 7 :

### Composition sous forme de bâtonnet (stick) :

| Huile de ricin | qsp 100 % |
|---|---|
| Cires végétales | 5,0 - 15,0 % |
| Autres cires | 10,0 - 20,0 % |
| Extrait lipidique d'Andiroba | 1,0 - 25,0 % |
| Acétate de tocophérol | 0,1 % |
| Vaseline | 5,0 - 10,0 % |
| Beurre de karité | 5,0 % |
| Parfum | 0,3 % |

Les extraits lipidiques d'Andiroba peuvent être utilisés, en tant que cosmétique ou dans un but pharmaceutique, à des teneurs de 0,01 à 100 %.
De préférence, les compositions selon l'invention comprennent de 0,01 % à 25 % en poids, d'extrait lipidique d'Andiroba.

## Revendications

1. Utilisation d'un extrait lipidique d'Andiroba pour la préparation d'une composition pharmaceutique ou cosmétique destinée au traitement de la cellulite.

2. Utilisation d'un extrait lipidique d'Andiroba selon la revendication 1, **caractérisée en ce que** l'extrait lipidique d'Andiroba est obtenu à partir des amandes de *Carapa guinanensis*.

3. Utilisation d'un extrait lipidique d'Andiroba selon la revendication 2, **caractérisée en ce que** l'extrait lipidique d'Andiroba est obtenu par réduction en pâte des amandes, traitement à chaud de la pâte, passage sous presse et décantation puis éventuellement filtration.

4. Utilisation d'un extrait lipidique d'Andiroba selon la revendication 2, **caractérisée en ce que** l'extrait lipidique d'Andiroba est obtenu par extraction de la fraction huileuse de l'amande concassée, au moyen d'un solvant apolaire à une température inférieure à 60°C, suivie d'une élimination ultérieure du solvant.

5. Utilisation d'un extrait lipidique d'Andiroba selon l'une quelconque des revendications précédentes, dans une composition cosmétique ou pharmaceutique comprenant de 0,01 à 100 %, de préférence de 0,01 à 25 % en poids, d'extrait lipidique et un véhicule à usage cosmétique ou pharmaceutique.

**Claims**

1. Use of a lipid extract of andiroba for preparing a pharmaceutical or cosmetic composition for the treatment of cellulite.

2. Use of a lipid extract of andiroba according to Claim 1, **characterised in that** the lipid extract of andiroba is obtained from *Carapa guinanensis* kernels.

3. Use of a lipid extract of andiroba according to Claim 2, **characterised in that** the lipid extract of andiroba is obtained by reducing the kernels to a paste, subjecting the paste to a heat treatment, pressing and decanting it, and then optionally filtering it.

4. Use of a lipid extract of andiroba according to Claim 2, **characterised in that** the lipid extract of andiroba is obtained by extracting the oily fraction of the crushed seed by means of an apolar solvent at a temperature below 60 °C, followed by subsequent elimination of the solvent.

5. Use of a lipid extract of andiroba according to any one of the preceding claims in a cosmetic or pharmaceutical composition comprising 0.01 to 100 wt.%, preferably 0.01 to 25 wt.% of lipid extract and a vehicle for cosmetic or pharmaceutical use.

**Patentansprüche**

1. Verwendung eines Lipidextrakts von Andiroba zur Herstellung einer pharmazeutischen oder kosmetischen Zubereitung für die Zellulitebehandlung.

2. Verwendung eines Lipidextrakts von Andiroba nach Anspruch 1, **dadurch gekennzeichnet, dass** der Lipidextrakt aus Kernen von *Carapa guianensis* gewonnen wird.

3. Verwendung eines Lipidextrakts von Andiroba nach Anspruch 2, **dadurch gekennzeichnet, dass** der Lipidextrakt von Andiroba durch Zerkleinern der Kerne zu Brei, Hitzebehandeln des Breis, Pressen, Dekantieren und gegebenenfalls Filtrieren gewonnen wird.

4. Verwendung eines Lipidextrakts von Andiroba nach Anspruch 2, **dadurch gekennzeichnet, dass** der Lipidextrakt durch Extraktion der Ölfraktion der zerkleinerten Kerne mit einem unpolaren Lösungsmittel bei einer Temperatur unterhalb von 60°C, gefolgt von einem Entfernen des Lösungsmittels gewonnen wird.

5. Verwendung eines Lipidextrakts von Andiroba nach einem der vorhergehenden Ansprüche in einer kosmetischen oder pharmazeutischen Zubereitung, enthaltend 0,01 bis 100 Gew.%, vorzugsweise 0,01 bis 25 Gew.% Lipidextrakt und einen Träger zur kosmetischen oder pharmazeutischen Behandlung.